# EUROPÄISCHE PATENTANMELDUNG

(11) **EP 1 671 652 A1**
(43) Veröffentlichungstag der Anmeldung: **21.06.2006**
(21) Anmeldenummer: 05026557.8
(22) Anmeldetag: 06.12.2005
(51) Int. Cl.: A61K 47/12, A61K 31/722, A61P 17/04

(54) **Verwendung von Chitosan gegen Juckreiz**

(30) Priorität: 15.12.2004 DE 102004060246
(71) Anmelder: Cognis IP Management GmbH, 40589 Düsseldorf (DE)
(72) Erfinder: Pahmeier, Andrea, 15806 Zossen (DE); Hahnemann, Birger, 15806 Zossen (DE); Lötzbeyer, Thomas, 85386 Eching (DE); Scholz, Claudia, 12203 Berlin (DE)

(57) **Zusammenfassung**

Vorgeschlagen wird die Verwendung von Chitosanen und Chitosanderivaten zur Herstellung von topischen Zubereitungen gegen Juckreiz.

Vorzugsweise wird reines Chitosan mit höherem Molekulargewicht wie 300.000 bis 2.000.000 g/mol oder 500.000 bis 5.000.000 g/mol, sowie Mischungen unterschiedlicher Molekulargewichtsbereiche eingesetzt. Des Weiteren werden topische Zubereitungen gegen Juckreiz offenbart, die a) 0,001 bis 5 Gew. % Chitosan mit einem Molekulargewicht von 300.000 bis 2.000.000 g/mol, b) 0,001 bis 5 Gew. % Chitosan mit einem Molekulargewicht von 500.000 bis 5.000.000 g/mol und c) 0,01 bis 5 Gew. % Hydroxycarbonsäure aufweisen.

## Beschreibung

### Gebiet der Erfindung

Die Erfindung befindet sich auf dem Gebiet der Kosmetik und Pharmazie und betrifft die Verwendung von Chitosan und seinen Derivaten zur Herstellung von Medikamenten gegen Juckreiz.

### Stand der Technik

Erkrankungen der Haut sind sehr oft mit quälendem Juckreiz begleitet. Häufige Ursachen sind Ekzeme, entzündliche Hautveränderungen die mit Rötungen, Schwellungen, Bläschen, Schuppung usw. verbunden sein können. Das optische Erscheinungsbild führt mitunter zur sozialen Isolation der Betroffenen, wenn durch Kratzen Sekundärinfektionen ausgelöst werden. Zudem führt Juckreiz oft zu Schlafstörungen und -insbesondere im Fall von Kindern - zu extremen Belastung der gesamten Familie.
Juckende Dermatosen verlaufen phasenhaft: In der akuten Phase (Schub) überwiegen Rötung, Überwärmung und Brennen, teilweise verbunden mit Hautnässen. Es besteht das Bedürfnis nach Kühlung. In der chronischen Phase zwischen den Schüben überwiegt die Austrocknung, Schuppenbildung und starker Juckreiz. Eine größe Gruppe von Patienten ist resistent gegen alle gängige Therapien.
Ein weiteres Problem ergibt sich aus dem Teufelskreis von Juckreiz und Infektionen. Bedingt durch Juckreiz erfolgt starkes Kratzen, welches Hautläsionen bedingen kann, die durch bakterielle Infektionen erneut Juckreiz auslösen können, denn derartige Infektionen führen zur Ausschüttung von Mediatoren, die wiederum den Juckreiz verstärken. So erfolgt ein Ringschluss. In der Phase der Wundheilung tritt dann erneut ein starker Juckreiz auf, gefolgt von intensivem Kratzen und der Kreis startet erneut. Die meisten medikamentösen Therapien sind auf Grund von Nebenwirkungen als Langzeittherapie ungeeignet.

Je nach Ursache unterscheidet man zwischen endo- und exogenen Ekzemen. Oftmals ist eine umfangreiche Diagnostik erforderlich, um den pruritogenen Stimulus zu identifizieren.
Endogen bedingtem Juckreiz können die unterschiedlichsten Erkrankungen zu Grunde liegen, wie beispielsweise Nahrungsmittelallergien, Stoffwechselerkrankungen, hormonell bedingte Störungen (Leber- bzw. Nierenerkrankungen, Schilddrüsenüberfunktion), Krebs, Erkrankungen des Blutes. Exogene Einflüsse zur Juckreizauslösung sind zum Beispiel Kontaktallergien, Sonnenbrand, Parasitenbefall und Insektenstiche. Juckreiz ist oft auch eine Folge der altersbedingten Austrocknung der Haut.
Generalisierter Juckreiz weist häufig auf eine medikamentöse oder tumorbedingte Genese hin, während ein lokalisierter Juckreiz sowohl bei Infektionskrankheiten der Haut als auch beim atopischen Ekzem (Neurodermitis) auftritt. Atopiker leiden grundsätzlich unter einer gestörten Hautbarriere und sind im Gegensatz zu Nichtatopikern mit Staphylococcus aureus kolonisiert. Diese Keime spielen nicht nur bei der Infektion eine Rolle, sondern auch in der Unterhaltung der entzündlichen Reaktionen. Staphylococcus aureus ist bekannt für die Sekretion bakterieller Superantigene. Hierbei handelt es sich um eine Gruppe hochmolekularer Proteine. Sie induzieren die Sekretion von IFN-γ, IL-1 und TNF-α und resultieren in einem komplexen Entzündungsgeschehen.

Das Phänomen Juckreiz biochemisch zu erklären ist bis heute nicht möglich, da die zugrunde liegenden Mechanismen sehr komplex sind, Auslöser bislang noch nicht eindeutig identifiziert wurden und das Netzwerk der Wirkweisen noch ungeklärt ist. Als Auslöser werden verschiedene Juckreizmediatoren diskutiert wie beispielsweise Histamin, verschiedene Neuropeptide, Prostaglandine, Serotonin, Acetylcholin und Bradykinin. Auch psychologischer Stress kann die Störung der physiologischen Balance der Haut auslösen, die in Juckreiz und damit verbundenen entzündlichen Prozessen mündet.
Die US-Patentanmeldung **US 2003/0039669 A1** offenbart die Verwendung von Komplexbildnern in topischen Zubereitungen zur Reduktion von Symptomen, die mit einer Histaminfreisetzung verbunden sind, insbesondere von Juckreiz und Erythembildung. Neben EDTA und einer Vielzahl weiterer Komplexbildner wird auch Chitosan als ein möglicher Histamin-Komplexbildner genannt. Histamin ist allerdings nur ein Faktor von vielen der juckreizauslösenden Substanzen. Die Behandlung des Pruritus soll jedoch nicht auf die Unterdrückung der durch Histaminausschüttung erzeugten Juckreizbildung beschränkt sein.

Die Behandlung des Juckreizes ist somit so vielfältig wie seine möglichen Entstehungswege.

Es werden derzeit verschiedene therapeutische Ansätze verfolgt: Die topische Behandlung mit kühlenden Cremes und Lotionen, Corticosteroiden, Capsaicin, Antihistaminika, Doxepin und Tacrolismus. Systemisch kann die Behandlung mit Antihistaminika, Corticosteroiden, Opiatantagonisten, Antidepressiva, Cyclosporin oder Phototherapie erfolgen (Wassilew S. Juckreiz: eine diagnostische und therapeutische Crux ; Deutsches Ärzteblatt Jg.99, April 2002). Ein abgestuftes therapeutisches Konzept zur Behandlung von Juckreiz existiert nicht, da die Intensität des Juckreizes schlecht bestimmt werden kann. Die Behandlung mit Corticosteroiden wird jedoch von vielen Patienten abgelehnt.

Die Verwendung von Chitosan im medizinischen und pharmazeutischen Bereich aufgrund seiner antimikrobiellen und entzündungshemmenden Eigenschaften ist lange bekannt.
In der deutschen Offenlegungsschrift **DE 101 58 712 A1** wurde eine Zubereitung, die eine Mischung aus photosynthetisch arbeitenden Mikroorganismen und Leuchtbakterien, sowie ein Polysaccharid auf Chitinbasis enthält zur Behandlung von Veränderungen der Haut beschrieben. Zu diesen Veränderungen zählt neben Pilz- und Bakterienerkrankungen auch indifferentes allgemeines Hautjucken. Erfindungswesentlich soll gerade die Kombination der Mischung aus Mikroorganismen, Leuchtbakterien und dem Polysaccharid auf Chitinbasis sein, um die erkrankte Hautoberfläche effektiv zu behandeln.

Die Aufgabe der vorliegenden Erfindung hat darin bestanden, Mittel mit verbesserter juckreizstillender Wirksamkeit zur Verfügung zu stellen, die sich durch eine besondere Verträglichkeit auszeichnen, wenig Nebenwirkungen aufweisen, nicht zur Resistenzbildung neigen und daher zur Langzeittherapie gut geeignet sind. Es wird die Bereitstellung einer beruhigenden Substanz angestrebt, welche den Juckreiz über Stunden mindert öder ganz ausschaltet und zugleich die Eigenschaften der Haut verbessert, dabei jedoch den Organismus aufgrund seiner chemischen Eigenschaften nicht belastet.

### Beschreibung der Erfindung

Gegenstand der Erfindung ist die Verwendung von Chitosan und/oder Chitosanderivaten zur Herstellung von topischen Zubereitungen gegen Juckreiz.
Die juckreizstillende Wirkung konnte bei Chitosanen mit einem Molekulargewicht von 10.000 bis 5000.000 g/mol (gemessen durch wässrige Gelpermeationschromatographie, GPC kalibriert gegen Pullulan-Standard), überraschender Weise aber besonders bei höhermolekularen Chitosanen mit einem Molekulargewicht im Bereich von 300.000 bis 2000.000 g/mol sowie 500.000 bis 5000.000 g/mol , festgestellt werden. Die Wirkung tritt schnell ein und hält über mehrere Stunden an. Die Chitosanzubereitungen lindern den Juckreiz zwar ohne die zugrundeliegende Krankheit zu kurieren, aber sie haben eine nachgewiesene hautbefeuchtende und antiirritierende Wirkung, die zu einer Verbesserung der Hautbarriere beiträgt.
Die Verbesserung der Hautbarriere ist essentiell, da durch das Kratzen die Hautbarriere zerstört wird. Es wird somit verhindert, dass vermehrt Keime einwandern, die eine immunologische Reaktion initiieren, die wiederum zu einer vermehrten Ausschüttung von Mediatoren führen und damit den Juckreiz verstärken.
Die eingesetzten Chitosanqualitäten besitzen ebenfalls eine nachgewiesene antimikrobielle Wirkung und können daher gegen Infektionen durch eindringende Keime vorbeugen. Mit der Anwendung der beschriebenen Zubereitung kann der oben beschriebene Teufelskreis unterbrochen werden

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten: 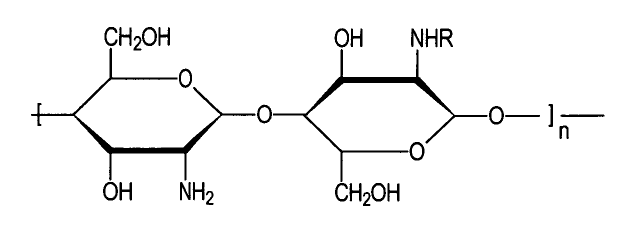 Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus der französischen Patentanmeldung FR 2701266 A bekannt. Vorzugsweise werden solche Typen eingesetzt, wie sie in den deutschen Patentanmeldungen **DE 4442987 A1** und **DE 19537001 A1** (Henkel) offenbart werden. Diese Chitosane weisen ein durchschnittliches Molekulargewicht von 10.000 bis 5.000 000 Dalton auf und zeichnen sich durch einen Deacetylierungsgrad im Bereich von 80 bis 88 %, vorzugsweise 82 bis 85 %, sowie einen Aschegehalt von weniger als 0,3 Gew.-%, vorzugsweise weniger als 0,1 Gew. % aus. Sie haben in der Regel eine Viskosität nach Brookfield (1 Gew.-%ig in Glycolsäure; Brookfield-Methode in einem RVF-Viskosimeter mit Spindel 5, 10 Upm)) unterhalb von 30.000 mPas.

Zur Optimierung der juckreizstillenden Wirkung ist das Molekulargewicht der Chitosane von entscheidender Bedeutung. Es können Chitosane mit einem mittleren Molekulargewicht von 10.000 bis 5.000.000 g/mol (gemessen durch wässrige Gelpermeationschromatographie, GPC kalibriert gegen Pullulan-Standard) eingesetzt werden. Eine bessere juckreizstillende Wirkung stellt man jedoch bei Chitosanen höheren Molekulargewichtes fest, in einer bevorzugten Ausführung werden daher Chitosane mit einem Molekulargewicht von 500.000 bis 5.000.000 g/mol (Handelsprodukt: Hydagen® CMFP und CMF sowie Chitopharm® L der Firma Cognis Deutschland GmbH& Co. KG) eingesetzt, besonders bevorzugt sind Chitosane mit einem Molekulargewicht im Bereich von 300.000 bis 2.000.000 g/mol (Handelsprodukt Hydagen® DCMF sowie Chitopharm® M der Firma Cognis Deutschland GmbH& Co. KG).
Die Chitosane werden bevorzugt in wässriger Lösung in einer Konzentration von 0,001 bis 5 Gew. % bezogen auf die Formulierung, bevorzugt 0,01 bis 3 Gew. %, besonders bevorzugt 0,1 bis 1,5 Gew. % und speziell 0,5 bis 0,8 Gew. % formuliert.
Da Chitosanqualitäten unterschiedlicher Molekulargewichte auch ein unterschiedliches Wirksamkeitsspektrum gegen mikrobielle Keime - insbesondere gegen Staphylokokken und/oder Pseudomonaden - aufweisen, hat es sich bewährt, in der juckreizstillenden Formulierung diese Verteilungen zu kombinieren, um gleichzeitig ein möglich breites antimikrobielles Wirkspektrum zu erzielen. Besonders bevorzugte Anwendungslösungen enthalten daher Chitosanqualitäten mit einem Molekulargewicht von 500.000 bis 5.000.000 g/mol und mit einem Molekulargewicht von 300.000 bis 2.000.000 g/mol in einer Verteilung von 10 : 90 bis 90 : 10, vorzugsweise 40 : 60 bis 60 : 40 und besonders bevorzugt 50 : 50.

In einer besonderen Ausführungsform werden Chitosane mit einem Molekulargewicht von 50.000 bis 1000.000 g/mol (Handelsprodukt Hydagen® HCMF sowie Chitopharm® S der Firma Cognis Deutschland GmbH& Co. KG) in der Lösung der höhermolekularen Chitosane eingesetzt, um die Viskosität der Lösungen auf den gewünschten Grad einzustellen. Zu diesem Zweck wird eine Lösung von 0,1 bis 0,5 Gew. % in die Lösung der anderen Verteilungen bis zum Einstellen der gewünschten Qualität eingetropft.

Diese nach dem Verfahren der **DE 4442987 A1** erhältlichen Chitosane stellen Chitosane mit höherem Molekulargewicht dar.

Eingesetzt werden auch sogenannte Oligochitosane, auch Oligoglucosamine genannt, bei denen es sich um Chitosane mit geringem Molekulargewicht handelt. Diese werden in einem zweiten Schritt unter dem Einfluss von Säuren weiter abgebaut und die Spaltprodukte, also die Oligoglucosamine, die jetzt ein Molekulargewicht von 500 bis 5.000 und vorzugsweise 800 bis 1.500 aufweisen einer Membranfiltration unterworfen, um sie von Verunreinigungen und insbesondere von Salzen zu befreien. Diese Oligochitosane haben neben der antimikrobiellen und anti-inflammatorischen Wirksamkeit zusätzlich regenerative und wachstumsstimulierende Eigenschaften und können bevorzugt zur Behandlung von Wunden eingesetzt werden, die mit Juckreiz begleitet sind.

Neben den Chitosanen kommen im Sinne der Erfindung auch derivatisierte Chitosane in Betracht, insbesondere die, bei denen der kationische Charakter durch die Derivatisierung erhalten bleibt. Dazu zählen Carboxylierungs-, Succinylierungs- oder Alkoxylierungsprodukte des Chitosans, wie sie beispielsweise in der deutschen Patentschrift DE-C2 3713099 (L'Oréal) sowie der deutschen Patentanmeldung DE-A1 196 04 180 (Henkel) beschrieben werden.
Das reine underivatisierte Chitosan hat sich jedoch am wirksamsten erwiesen hinsichtlich der juckreizstillenden Wirkung und seiner guten Hautverträglichkeit.

### Gewerbliche Anwendbarkeit

Das Chitosan und/oder die Chitosanderivate werden in die topischen Zubereitungen in Mengen von 0,001 bis 5 Gew.-%, bevorzugt 0,01 bis 3 Gew.-%, insbesondere 0,1 bis 1,5 Gew.-% und speziell 0,5 bis 0,8 Gew.-% bezogen auf die Gesamtformulierung eingearbeitet. Geeignete Formulierungen sind Lösungen, Emulsionen, Suspensionen, Gele, Cremes, Sprays und Fluids, da diese zusätzlich einen erhöhten Wassergehalt beinhalten können, der einerseits für die Lösung des Chitosans andererseits wegen seines zusätzlich kühlenden Effektes vorteilhaft ist.

Die Chitosane werden mit Zusatz von Mineralsäuren oder organischen Carbonsäuren in Lösung gebracht.
Als Mineralsäuren sind geeignet Salzsäure, Phosphorsäure, Salpetersäure sowie Schwefelsäure, als organische Carbonsäuren seien genannt: Ameisensäure, Milchsäure, Propionsäure, Maleinsäure, Brenztraubensäure, Glykolsäure, Bernsteinsäure, Essigsäure, Zitronensäure, Weinsäure, Oxalsäure, Bernsteinsäure und Adipinsäure, sowie auch aromatische Carbonsäuren, wie Benzoesäure oder Naphtoesäuren..
Besonders juckreizstillende Wirkung wies jedoch die Kombination mit Hydroxycarbonsäuren auf. Gut geeignet sind insbesondere Hydroxycarbonsäuren mit 2 bis 12 Kohlenstoffatomen. Es können ein oder mehrere Hydroxylgruppen sowie ein oder mehrere Carboxylgruppen im Molekül vorhanden sein. Beispiele sind Glycolsäure, Milchsäure, Äpfelsäure, Weinsäure sowie Zitronensäure, es können aber auch aromatische Hydroxycarbonsäuren verwendet werden wie z.B. Mandelsäure. Speziell bevorzugt wird hier die Kombination von Chitosan und Milchsäure, die sich nicht nur durch die gute juckreizstillende Wirkung, sondern auch durch eine besonders gute Regeneration der Hautbarriere von anderen Säuren abgrenzt. Außerdem wies Milchsäure die besten Verarbeitungseigenschaften auf.
Es werden die Mengen an Säure eingesetzt, die zu einer teilweisen bis vollständigen Lösung des Chitosans benötigt werden. Üblicherweise sind dies 10⁻⁴ bis 10⁻² mol Säuregruppen pro g Chitosan, insbesondere 1 - 3 × 10⁻³ mol Säuregruppen / g Chitosan.

Zur Entfernung überschüssiger Säure aus der zu verwendenden Lösung wird durch Zugabe einer Base der pH Wert der Lösung auf pH 1,0- 7,5 vorzugsweise 2,5- 6,5 insbesondere 3,0-5,0 eingestellt. Dadurch wird Chitosan in Lösung gehalten und kann seine juckreizlindende Wirkung entfalten. Es wurde beobachtet, dass der Anteil des gelösten Chitosans einen wesentlichen Einfluss auf die Juckreizminderung hat, so dass das Chitosan vorteilhafterweise in gelöster Form vorliegen soll.

Ein weiterer Gegenstand der Erfindung sind daher topische Zubereitungen zur Behandlung von Juckreiz, enthaltend
a) 0,001 bis 5 Gew. % Chitosan mit einem Molekulargewicht von 300.000 bis 2.000.000 g/mol.
b) 0,01 bis 5 Gew. % Hydroxycarbonsäure.
oder
a) 0,001 bis 5 Gew. % Chitosan mit einem Molekulargewicht von 500.000 bis 5.000.000 g/mol und
b) 0,01 bis 5 Gew. % Hydroxycarbonsäure.

Bevorzugte Rezepturen enthalten
a) 0,001 bis 5 Gew. % Chitosan mit einem Molekulargewicht von 300.000 bis 2.000.000 g/mol.
b) 0,001 bis 5 Gew. % Chitosan mit einem Molekulargewicht von 500.000 bis 5.000.000 g/mol und
c) 0,01 bis 5 Gew. % Hydroxycarbonsäure.

Besonders bevorzugte Rezepturen enthalten:
a) 0,001 bis 5 Gew. % Chitosan mit einem Molekulargewicht von 300.000 bis 2.000.000 g/mol.
b) 0,001 bis 5 Gew. % Chitosan mit einem Molekulargewicht von 500.000 bis 5.000.000 g/mol,
c) 0,01 bis 5 Gew. % Hydroxycarbonsäure und
d) 0,001 bis 1 Gew. % Chitosan mit einem Molekulargewicht von 50.000 bis 1.000.000 g/mol.
oder
a) 0,001 bis 3 Gew. % Chitosan mit einem Molekulargewicht von 300.000 bis 2.000.000 g/mol.
b) 0,001 bis 5 Gew. % Chitosan mit einem Molekulargewicht von 500.000 bis 5.000.000 g/mol,
c) 0,01 bis 5 Gew. % Hydroxycarbonsäure und
d) 0,01 bis 1 Gew. % Oligochitosane mit einem Molekulargewicht von 500 bis 5000 g/mol.

Speziell bevorzugte Mittel enthalten:
a) 0,1 bis 1,5 Gew. % Chitosan mit einem Molekulargewicht von 300.000 bis 2.000.000 g/mol.
b) 0,1 bis 1,5 Gew. % Chitosan mit einem Molekulargewicht von 500.000 bis 5.000.000 g/mol und
c) 0,1 bis 1,5 Gew. % Milchsäure.

Die erfindungsgemäßen topischen Zubereitungen zur Juckreizstillung zeichnen sich durch hohe Hautverträglichkeit aus und sind bei allen Krankheitsbildern und Hauterscheinungen, die Juckreiz hervorrufen können, wie beispielsweise Alterung der Haut, Sonnenbrand, trockene Haut, innere Erkrankungen auf belasteteten Haut- und Haarbereichen bei Menschen und auch Tieren zu verwenden.

Die beschriebenen Mittel aus Chitosan und Hydroxycarbonsäuren können direkt auf die Haut aufgetragen werden oder weiterverarbeitet werden zu Gelen, Salben, Cremes, Sprays, Fluids oder Emulsionen werden und dadurch in einer aufeinander abgestimmten Pflegeserie mit juckreizstillendem Charakter und vermehrt hautpflegenden Eigenschaften resultieren.

Dabei sind auch Kombinationen mit weiteren pflegenden Substanzen und Wirkstoffen vorteilhaft. Beispielsweise kann ein zweiteiliges Pflegekonzept in Form einer juckreizstillenden Notfallmedizin und einer hautpflegenden Mischung, die die Regeneration der Haut zwischen den "Juckreizanfällen" fördert.
So kann eine Chitosanhaltige Basislösung in O/W Emulsionen oder W/O-Emulsionen oder Gelen in Konzentrationen von 0,1 bis 70 Gew. %, vorzugsweise 1 bis 30 Gew.% und besonders bevorzugt 3 bis 15 Gew. % bezogen auf die Endformulierung leicht eingearbeitet werden. Die Chitosane können aber auch direkt der O/W Emulsion zugesetzt werden oder der Wasserphase beigemengt werden vor der Mischung der W/O Emulsion.

### Weitere Wirkstoffe

Weitere Wirkstoffe, die Juckreizstillende Wirkung aufweisen seien Lokalanästhetika wie Bencain, Mepivacain oder Lidocain, sowie das häufig eingesetzte Polidocanol, Menthol oder ätherische Öle.

Ebenfalls können Wirkstoffe eingesetzt werden, die eine Wundheilung beschleunigen wie beispielsweise Pflanzenextrakte wie Aloe vera, Antibiotika, Antiseptika, Antimykotika, Analgetika, Antihistaminika, Enzyminhibitoren, Glucocorticoide, Vitamine, Virustatika, Wachstumsfaktoren, Steroide, Enzyme oder Hormone.
Zur Behandlung komplexer Hauterkrankungen eingesetzt werden weitere Wirkstoffe wie Harnstoff, CLA, Phytosterole, Vitamine, Hyaluronsäure, pflegende Öle (Macadamiernu-ß¡öl, Sanddornöl, Mandelöl).

### Beispiele

Für weitere Formulierungen wurde eine spezielle Basislösung (1) hergestellt in dem
a) 0,75 Gew. % Chitosan mit einem Molekulargewicht von 300.000 bis 2.000.000 g/mol (Hydagen® DCMF Cognis Deutschland GmbH & Co. KG) durch Rühren bei Raumtemperatur in deionisiertem Wasser gelöst wurden, sowie in einer zweiten Lösung
b) 0,75 Gew. % Chitosan mit einem Molekulargewicht von 500.000 bis 5.000.000 g/mol (Hydagen® CMFP Cognis Deutschland GmbH & Co. KG) in deionisiertem Wasser gelöst wurden
Beide Lösungen a) und b) wurden 1:1 gemischt, so dass die Basislösung 0,75 Gew. % Chitosan enthielt. Dieser Lösung wurde 1 Gew. % Milchsäure zugesetzt.
Nach Filtration zur Abtrennung unlöslicher Bestandteile wurde überschüssige Säure durch Zugabe von Natriumhydroxidlösung entfernt und auf einen pH-Wert von pH 5 eingestellt.
Diese Formulierung trugen sich 3 Probanden auf Juckreiz-betroffene Hautflächen auf den Armen. Die juckreizstillende Wirkung hielt über 10 Stunden an.

Die Basislösung (1) wurde weiterverarbeitet und zu 15 Gew. % in eine O/W Emulsion der Zusammensetzung Lanette® N Gew. 10%, Eutanol® G Gew. 4 % und Paraffinöl Gew. 4 % sowie Glycerin Gew. 5% , Aqua purificata ad 100 eingearbeitet (2).
Die juckreizstillende Wirkung dieser Formulierung (2) hielt bei den gleichen Probanden über 2 Stunden.

Eine weitere Lösung (3) enthaltend
a) 0,75 Gew. % Chitosan mit einem Molekulargewicht von 50.000 bis 1.000.000 g/mol
   (Hydagen® HCMF Cognis Deutschland GmbH & Co. KG)
   und
b) 1 Gew. % Milchsäure
wurde durch Rühren des Chitosans bei Raumtemperatur in deionisiertem Wasser und Zufügen der Milchsäure hergestellt.
Diese Lösung (3) trugen sich die gleichen Probanden auf die Juckreiz-betroffene Hautflächen auf den Armen. Die juckreizstillende Wirkung der Lösung (3) war jedoch nur minimal. Ein Zeitpunkt des Ausklingens der Wirkung konnte von keinem Probanden angegeben werden.

## Patentansprüche

1. Verwendung von Chitosan und/oder Chitosanderivaten zur Herstellung von topischen Zubereitungen gegen Juckreiz.

2. Verwendung gemäß Anspruch 1, **dadurch gekennzeichnet, dass** das Chitosan und/oder die Chitosanderivate ein Molekulargewicht von 10.000 bis 5.000000 g/mol aufweisen.

3. Verwendung gemäß Anspruch 1, **dadurch gekennzeichnet, dass** das Chitosan und/oder die Chitosanderivate ein Molekulargewicht von 500.000 bis 5.000000 g/mol aufweisen.

4. Verwendung gemäß Anspruch 1, **dadurch gekennzeichnet, dass** das Chitosan und/oder die Chitosanderivate ein Molekulargewicht von 300.000 bis 2.000000 g/mol aufweisen.

5. Verwendung gemäß Anspruch 1, **dadurch gekennzeichnet, dass** Mischungen von reinem Chitosan mit zwei unterschiedlichen Molekulargewichtsverteilungen eingesetzt werden, vorzugsweise Mischungen der Molekulargewichtsbereiche 300.000 bis 2.000000 g/mol und 500.000 bis 5.000000 g/mol.

6. Verwendung gemäß den Ansprüchen 1 bis 5, **dadurch gekennzeichnet, dass** das Chitosan und/oder die Chitosanderivate in einer Menge von 0,001 bis 5 Gew. % in den topischen Zubereitungen vorliegen.

7. Verwendung gemäß den Ansprüchen 1 bis 6, **dadurch gekennzeichnet, dass** das Chitosan und/oder die Chitosanderivate in Kombination mit Hydroxycarbonsäuren eingesetzt werden.

8. Verwendung gemäß den Ansprüchen 1 bis 7, **dadurch gekennzeichnet, dass** als Chitosane oder Chitosanderivate kationisch derivatisierte Chitosane oder Oligoglucosamine eingesetzt werden.

9. Verwendung gemäß den Ansprüchen 1 bis 8, **dadurch gekennzeichnet, dass** die topischen Zubereitungen in Form von Lösungen, Emulsionen, Suspensionen, Gelen, Cremes, Sprays oder Fluids vorliegen.

10. Topische Zubereitungen zur Behandlung von Juckreiz, enthaltend
a) 0,001 bis 5 Gew. % Chitosan mit einem Molekulargewicht von 300.000 bis 2.000.000 g/mol.
b) 0,001 bis 5 Gew. % Chitosan mit einem Molekulargewicht von 500.000 bis 5.000.000 g/mol und
c) 0,01 bis 5 Gew. % Hydroxycarbonsäure.
